# EUROPEAN PATENT SPECIFICATION

(11) **EP 4 580 507 B1**
(45) Date of publication and mention of the grant of the patent: **11.02.2026**
(21) Application number: 23771777.2
(22) Date of filing: 31.08.2023
(51) Int. Cl.: G16H 15/00, G16H 30/20, G16H 40/67, A61B 8/00, G16H 30/40, G16H 80/00, G16H 40/63

(54) **ULTRASOUND EXAM TRACKING**
ULTRASCHALLUNTERSUCHUNGSVERFOLGUNG
SUIVI D'EXAMEN PAR ULTRASONS

(30) Priority: 01.09.2022 US 202263403112 P
(43) Date of publication of application: 09.07.2025
(73) Proprietor: Koninklijke Philips N.V., 5656 AG Eindhoven (NL)
(72) Inventor: ERRICO, Claudia, 5656 AG Eindhoven (NL); BHARAT, Shyam, 5656 AG Eindhoven (NL); MANKOVICH, Gabriel Ryan, 5656 AG Eindhoven (NL)
(74) Representative: Philips Intellectual Property & Standards
(86) International application number: PCT/EP2023/073861
(87) International publication number: WO 2024/047143

(56) References cited:
- WO-A1-2021/259739
- WO-A2-2022/144177
- US-A1- 2003 097 054
- US-A1- 2008 201 350

## Description

### TECHNICAL FIELD

The present disclosure pertains to devices, systems and methods for capturing, storing and conveying information related to ultrasound probe manipulation performed during an imaging exam. Implementations involve determining probe positions and orientations based on image frames acquired during an ultrasound exam and logging that information together with discrete user inputs received by the imaging system during the exam to generate a comprehensive record of user performance.

### BACKGROUND

Log files generated during ultrasound imaging exams provide insightful information regarding exam workflow and efficiency. In ultrasound imaging, every button and keystroke pushed by the sonographer may be logged and stored in a workflow and utilization script. Each single exam may thus be recorded as a sequence of events collectively capturing the workflow narrative of the scan from beginning to end. Ongoing log file analysis can be used to modify the layout of ultrasound systems for improved workflow and to design customizable, dynamic keyboards and touch screens that mimic user preferences. The same analysis may inform modifications to underlying scan protocols that require unnecessary and inefficient user operation.

While usage information captured and stored in log files has proven valuable in shaping system and protocol design, additional improvements are needed to accurately monitor and record sonographer performance in a manner capable of enhancing user feedback and augmenting current data capture techniques.

WO 2021/259739 A1 relates to an adaptable user interface for a medical imaging system which can adapt automatically based on prior usage data of one or more users of the user interface, such that the functions, the layout or the appearance of user controls of the user interface may be changed.

### SUMMARY

The invention is defined by the independent claims. Advantageous embodiments are provided in the dependent claims.

The present disclosure describes devices, methods and systems configured to capture and store information related to ultrasound probe manipulations performed by a user during an ultrasound exam. Probe manipulation data, which may include underlying probe positions and orientations, may be determined using image frames acquired during an ultrasound exam. In particular, the image frames may be input to a neural network trained to estimate the associated probe position/orientation used to acquire the image frames based on landmarks identified within the frames. The probe manipulation data may be documented in log files together with documented user inputs, e.g., button pushes and keystrokes, typically received by an imaging system during an exam. By pairing probe manipulation data with user inputs and standard views associated with selected imaging protocols, a comprehensive record of user performance and exam workflow may be generated. Embodiments may also convert the comprehensive record embodied by the log files into narrative text descriptions provided to sonographers, lab managers, and radiologists for review.

In accordance with embodiments of the present disclosure, an ultrasound imaging system may include an ultrasound probe configured to acquire echo signals responsive to ultrasound pulses transmitted toward a subject during an ultrasound. The system may also include an input of pre-defined user inputs associated with an exam protocol that is similar to the ultrasound exam. The system may also include one or more processors in communication with the ultrasound probe. The processors may be configured to determine and track manipulations of the ultrasound probe performed during the ultrasound exam based on image frames generated from the echo signals; identify meaningful user inputs received during the ultrasound exam indicative of adherence to the exam protocol; and generate log files documenting the manipulations and meaningful user inputs.

In some examples, the meaningful user inputs may include an adjustment in at least one image acquisition setting comprising one or more of: a gain, a scan depth, an image contrast level, a color scale, a probe type, a frequency, or an imaging mode. In some examples, the meaningful user inputs may include one or more of: an image snapshot, a label, or a measurement of one or more features present in the image frames. In some examples, the manipulations of the ultrasound probe comprise changes in probe position and orientation.

In some examples, the one or more processors may be configured to determine and track manipulations of the ultrasound probe by inputting the image frames to a neural network. The neural network may be trained to identify at least one landmark present in one or more of the image frames and determine one or more ultrasound probe orientations and/or positions used to obtain the one or more image frames based on the at least one landmark identified therein. In some examples, the at least one landmark comprises one or more of: an anatomical feature, an image artifact, a medical instrument, or a transducer marker. In some examples, the one or more processors may be configured to identify meaningful user inputs received during the ultrasound exam indicative of adherence to an exam protocol by comparing a total list of actual user inputs to a list of expected user inputs associated with the exam protocol.

In some examples, the one or more processors may be configured to generate log files documenting the manipulations and meaningful user inputs by generating a first log file documenting the meaningful user inputs, generating a second log file documenting the manipulations of the ultrasound probe, and synchronizing the first log file with the second log file. In some examples, the one or more processors may be configured to generate log files documenting the manipulations and meaningful user inputs by generating a first log file documenting the meaningful user inputs, generating one or more object events documenting the manipulations of the ultrasound probe, and inserting the one or more object events into the first log file based on a unique timestamp associated with each of the one or more object events.

In some examples, the one or more processors may be further configured to generate narrative text describing the manipulations and meaningful user inputs documented in the log files. In some examples, the one or more processors may be further configured to determine and track manipulations of the ultrasound probe based input received from an external sensor monitoring movements of a user handling the ultrasound probe. In some examples, the one or more processors may be configured to determine and track manipulations of the ultrasound probe by inputting the input received from the external sensor to a neural network. The neural network may be configured to identify one or more of a relative position, orientation, or angle between two or more anatomical features of the user, and determine one or more ultrasound probe orientations and/or positions corresponding to the at least one relative position or angle.

In accordance with embodiments of the present disclosure, a method of ultrasound imaging may involve acquiring echo signals responsive to ultrasound pulses transmitted into a target region of a subject by an ultrasound probe during an ultrasound exam, receiving input of pre-defined user inputs associated with an exam protocol that is similar to the ultrasound exam, determining and tracking manipulations of the ultrasound probe performed during the ultrasound exam based on image frames generated from the echo signals, identifying meaningful user inputs received during the ultrasound exam indicative of adherence to the exam protocol, and generating log files documenting the manipulations and meaningful user inputs.

In some examples, the meaningful user inputs may include an adjustment in at least one image acquisition setting comprising one or more of: a gain, a scan depth, an image contrast level, a color scale, a probe type, a frequency, or an imaging mode. In some examples, the manipulations of the ultrasound probe may include changes in probe position and orientation. In some examples, determining and tracking manipulations of the ultrasound probe may involve identifying at least one landmark present in one or more of the image frames and determining one or more ultrasound probe orientations and/or positions used to obtain the one or more image frames based on the at least one landmark identified therein. In some examples, the at least one landmark comprises one or more of: an anatomical feature, an image artifact, a medical instrument, or a transducer marker.

In some examples, generating log files documenting the manipulations and meaningful user inputs may involve generating a first log file documenting the meaningful user inputs, generating a second log file documenting the manipulations of the ultrasound probe, and synchronizing the first log file with the second log file. In some examples, generating log files documenting the manipulations and meaningful user inputs may involve generating a first log file documenting the meaningful user inputs, generating one or more object events documenting the manipulations of the ultrasound probe, and inserting the one or more object events into the first log file based on a unique timestamp associated with each of the one or more object events.

Any of the methods described herein, or steps thereof, may be embodied in a non-transitory computer-readable medium comprising executable instructions, which when executed may cause one or more hardware processors to perform the method or steps embodied herein.

### BRIEF DESCRIPTION OF THE DRAWINGS

**FIG. 1** is a block diagram illustrating an example of an ultrasound system configured for comprehensive tracking and documentation of user performance during imaging exams in accordance with embodiments of the present disclosure.
**FIG. 2** is a block diagram of a collection of computational modules that may be implemented in accordance with embodiments of the present disclosure.
**FIG. 3** is a series of ultrasound images analyzed in accordance with embodiments of the present disclosure.
**FIG. 4** is a collection of example graphics representing the utilization of an external sensor in accordance with embodiments of the present disclosure.
**FIG. 5** is an example record of user inputs received during an ultrasound imaging exam in accordance with embodiments of the present disclosure.
**FIG. 6** is a representation of abbreviated log files that may be generated in accordance with embodiments disclosed herein.
**FIG. 7** is a schematic representation of the generation and use of workflow log files documenting ultrasound system usage and probe manipulation to generate narrative exam summaries in accordance with embodiments disclosed herein.
**FIG. 8** is a block diagram illustrating an example processor implemented in accordance with embodiments of the present disclosure.
**FIG. 9** is a block diagram illustrating additional components of an ultrasound system configured for comprehensive tracking and documentation of user performance during imaging exams in accordance with embodiments of the present disclosure.
**FIG. 10** is a flowchart of a method implemented in accordance with at least one embodiment of the present disclosure.

### DETAILED DESCRIPTION

Various embodiments are described more fully below with reference to the accompanying drawings, which form a part hereof, and which show specific exemplary embodiments. However, embodiments may be implemented in many different forms and should not be construed as limited to the embodiments set forth herein; rather, these embodiments are provided so that this disclosure will be thorough and complete, and will fully convey the scope of the embodiments to those skilled in the art. Embodiments may be practiced as methods, systems, or devices. Accordingly, embodiments may take the form of a hardware implementation, an entirely software implementation, or an implementation combining software and hardware aspects. The following detailed description is, therefore, not to be taken in a limiting sense.

Reference in the specification to "one embodiment" or to "an embodiment" means that a particular feature, structure, or characteristic described in connection with the embodiment is included in at least one embodiment of the invention. The appearance of the phrase "in one embodiment" in various places in the specification are not necessarily all referring to the same embodiment.

As used herein, "users" may include various medical professionals, clinicians, and personnel, non-limiting examples of which can include sonographers.

As used herein, "user inputs" may refer to discrete manual inputs received by an ultrasound imaging system before, during, and/or after an imaging exam. The user inputs may be implemented to perform a variety of actions necessary for exam execution, such as taking snapshots of images containing certain features, labelling various features, measuring certain features, indicating completion of one or more steps set forth in an exam protocol, initiating ultrasound image acquisition, and/or adjusting various image acquisition parameters, e.g., image gain, scan depth, image contrast, image brightness, color scale, imaging mode, etc. Non-limiting examples of discrete user inputs may include button pushes, keystrokes, trackball adjustments, switch actuations, touchscreen engagements, etc.

As used herein, "probe manipulation" may refer to adjustments in probe position and orientation performed by a user during an ultrasound exam, including translational and rotational movement. Probe manipulation may be defined relative to a stationary subject being imaged.

As used herein, "subject" may refer to a patient receiving outpatient or inpatient medical care, which may involve medical treatment or examination. The term "subject" is not limited to medical patients; it may also refer to a human or animal being imaged by an imaging system for a variety of reasons in a variety of settings.

Some portions of the description that follow are presented in terms of symbolic representations of operations on non-transient signals stored within a computer memory. These descriptions and representations are the means used by those skilled in the data processing arts to most effectively convey the substance of their work to others skilled in the art. Such operations typically require physical manipulations of physical quantities. These quantities may take the form of electrical, magnetic, or optical signals capable of being stored, transferred, combined, compared and otherwise manipulated. It is convenient at times, primarily for reasons of common usage, to refer to these signals as bits, values, elements, symbols, characters, terms, numbers or the like. Furthermore, it is also convenient at times, to refer to certain arrangements of steps requiring physical manipulations of physical quantities as modules or code devices, without loss of generality.

However, all of these and similar terms are to be associated with the appropriate physical quantities and are merely convenient labels applied to these quantities. Unless specifically stated otherwise as apparent from the following discussion, it is appreciated that throughout the description, discussions utilizing terms such as "processing," "computing," "calculating," "determining," "displaying" or the like, refer to the action and processes of a computer system, or similar electronic computing device, that manipulates and transforms data represented as physical electronic quantities within the computer system memories or registers or other such information storage, transmission, or display devices.

Certain aspects of the present invention include process steps and instructions that could be embodied in software, firmware, or hardware, and when embodied in software, could be downloaded to reside on and be operated from different platforms used by a variety of operating systems. Embodiments can comprise one or more applications available over the Internet, e.g., software as a service (SaaS), accessible using a variety of computer devices, e.g., smartphones, tablets, desktop computers, etc.

The present invention also relates to at least one apparatus configured to perform one or more of the operations disclosed herein. This apparatus may be specially constructed for the required purposes, or it may comprise a general purpose computer selectively activated or reconfigured by a computer program stored in the computer. Such a computer program may be stored in a computer readable storage medium, non-limiting examples of which may include read-only memories (ROMs), random access memories (RAMs), EPROMs, EEPROMs, magnetic or optical cards, application specific integrated circuits (ASICs), optical disks, CD-ROMs, floppy disks, magnetic-optical disks, or any type of media suitable for storing electronic instructions, and each coupled to a computer bus. Furthermore, the computers referred to herein may include a single processor or may be architectures employing multiple processor designs for increased computing capability.

An ultrasound system according to the present disclosure may implement one or more neural networks, for example at least one deep neural network (DNN), convolutional neural network (CNN) or the like, which may be uniquely synced with data acquisition hardware and at least one user interface. Example systems may utilize at least one neural network to identify various anatomical and/or artificial features appearing in acquired ultrasound images, and output corresponding ultrasound probe positions/orientations. Analysis of the neural network outputs on a frame-by-frame, frame stack and/or cine loop basis may be utilized to determine probe manipulations in the form of changes in probe position and/or orientation.

In various examples, the neural network(s) may be trained using any of a variety of currently known or later developed machine learning techniques to obtain a neural network (e.g., a machine-trained algorithm or hardware-based system of nodes) configured to analyze input data in the form of ultrasound image frames and identify certain features, such as anatomical features, ultrasound artifacts (e.g., A- or B-lines), medical equipment, etc.

Neural networks may provide an advantage over traditional forms of computer programming algorithms in that they can be generalized and trained to recognize data set features by analyzing data set samples rather than by reliance on specialized computer code. By presenting appropriate input and output data to a neural network training algorithm, neural network(s) of an ultrasound system according to the present disclosure can be trained to identify a plurality of anatomical and/or artificial features present within ultrasound image frames and output an indication of the probe position and/or orientation utilized to acquire the image frames.

The neural networks utilized according to the present disclosure may be hardware- (e.g., neurons are represented by physical components) or software-based (e.g., neurons and pathways implemented in a software application), and can use a variety of topologies and learning algorithms for training the neural network to produce the desired output. For example, a software-based neural network may be implemented using a processor (e.g., single or multi-core CPU, a single GPU or GPU cluster, or multiple processors arranged for parallel-processing) configured to execute instructions, which may be stored in computer readable medium, and which when executed cause the processor to perform a machine-trained algorithm.

An ultrasound system may include a display or graphics processor, which is operable to arrange the ultrasound image and/or additional graphical information, which may include a worklist of features to be imaged and/or measured, along with previously measured features, feature labels, annotations, tissue information, patient information, indicators, and other graphical components, in a display window for display on a user interface of the ultrasound system. In some embodiments, the ultrasound images, received user inputs, probe manipulation data, and/or log files documenting the same may be provided to a storage and/or memory device, such as a picture archiving and communication system (PACS) for reporting purposes, user guidance instruction, developmental progress tracking, or future machine training (e.g., to continue to enhance the performance of the neural network). In some examples, ultrasound images obtained during a scan may be selectively or automatically transmitted, e.g., over a communications network, to a specialist trained to interpret the information embodied in the images, e.g., a radiologist, an ultrasound specialist, a physician, or other clinician.

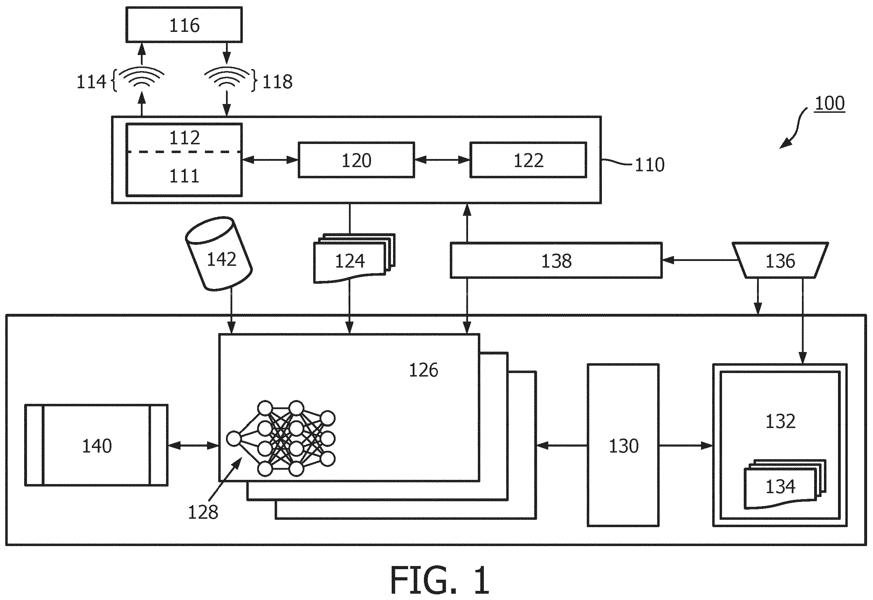

**FIG. 1** shows an example ultrasound system 100 according to embodiments of the present disclosure. As shown, the ultrasound system 100 may include an ultrasound data acquisition unit 110 comprising an ultrasound probe 111, which includes an ultrasound transducer array 112 configured to transmit ultrasound pulses 114 into a region 116 of a subject, e.g., abdomen, and receive ultrasound echoes 118 responsive to the transmitted pulses. As further shown, the ultrasound data acquisition unit 110 can include a beamformer 120 and a signal processor 122, which can be configured to generate a stream of discrete ultrasound image frames 124 from the ultrasound echoes 118 received at the array 112. The system can also include at least one data processor 126, e.g., a computational module or circuity, which may be configured to implement at least one deep neural network 128.

The neural network 128 may be configured to receive the image frames 124 directly from the signal processor 122 or via the data processor 126 and determine a corresponding view embodied by each frame, along with the position and/or orientation of the ultrasound probe 111 likely used to acquire it. The neural network 128 may operate in real or substantially real time during an ultrasound exam by recognizing one or more features or markers present in the image frames 124 on a frame-by-frame basis. In embodiments, the neural network 128 may also operate on a frozen buffer basis or pursuant to a review study. By analyzing the output of the neural network 128 in the context of a selected scan protocol, the data processor 126 may be configured to determine user manipulation of the probe 111 in the form of probe motion, e.g., changes in probe position and orientation, relative to the region 116 of the subject being imaged. In some embodiments, two or more neural networks 128 may be implemented by the data processor 126. Examples may include neural networks trained specifically to analyze certain types of image frames and/or image frames generated in accordance with particular exam types.

In some examples, the system 100 also includes a display processor 130 coupled with the data processor 126 and a graphical user interface 132. The display processor 130 may link the neural network 128 and data processor 126 to the user interface 132, thereby enabling the outputs generated by one or both components to be displayed on the user interface 132. The user interface 132 may be configured to display ultrasound images 134 of the region 116 in real or substantially real time as an ultrasound exam is being performed. Additional graphics displayed on the user interface 132 may include anatomical measurements obtained by the system and/or user, labeled anatomical and/or artificial features visible in the acquired image frames, bounding boxes generated around particular features, etc.

The user interface 132 may also be configured to receive various user inputs 136 at any time before, during, and/or after an ultrasound exam. As noted above, the user inputs 136 may comprise discrete manual inputs, e.g., button pushes or keystrokes, implemented to perform an ultrasound exam. With respect to the user interface 132, such inputs may comprise tapping or dragging various displayed icons. Additionally, the user inputs 136 may include user requests to view the acquisition parameters and/or probe manipulations implemented to obtain a given image. Such inputs may comprise manual clicking, tapping, or otherwise selecting an image of particular interest displayed on the user interface 132 during or after an ultrasound exam. In addition, the system 100 may include at least one hardware component 138, e.g., keyboard, mouse, trackball, etc., configured to receive user inputs 136 and coupled to the data processor 126 and acquisition unit 110.

As further shown, the system 100 may include a memory 140 configured to store a variety of data, such as neural network output, data processor output, image frames, etc. The memory 140 may also store log files generated during and after an ultrasound exam. The log files may include information documenting timestamped user inputs, e.g., button pushes, along with corresponding probe positions/orientations, probe manipulations, landmarks identified by the neural network, and image views related thereto.

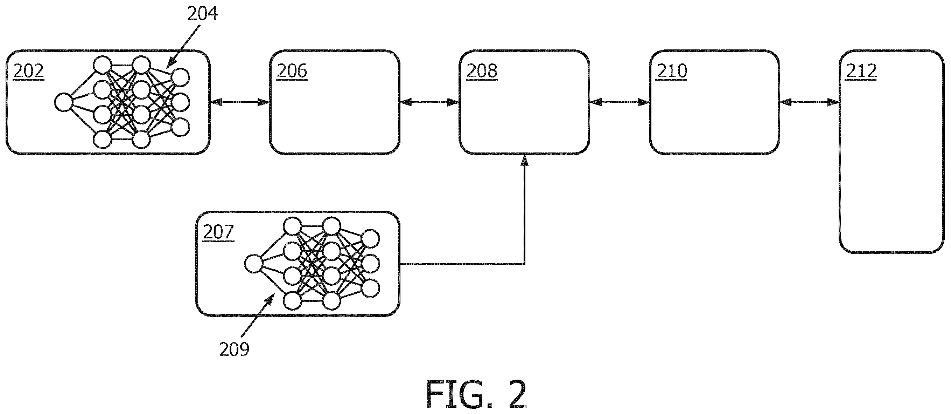

In some embodiments, the system 100 may additionally or alternatively include an external sensor 142 configured to detect and monitor movements of the user handling the ultrasound probe 111. The external sensor 142 may comprise a camera that may be mounted in an exam room. The data processor 126 may be communicatively coupled with the external sensor 142 and configured to determine manipulations of the probe 111 using the corresponding movements of the user detected by the sensor 142. As further set forth below in connection with FIG. 2, one or more additional neural networks may be trained to output probe positions/orientations/motions using inputs received from the sensor 142. The sensor 142 may be omitted in various examples. In others, the sensor 142 may replace or augment the image-based operations of neural network 128.

The configuration of the system 100 shown in **FIG. 1** may vary. For example, at least part of the system 100 may be portable or stationary. Various portable devices, e.g., laptops, tablets, smart phones, or other handheld devices may be used to implement one or more functions of the system 100. In embodiments that incorporate such devices, the ultrasound probe 111 may be connectable via USB interface. A variety of transducer arrays may be used, e.g., linear arrays, convex arrays, or phased arrays. The number and arrangement of transducer elements included in the sensor array 112 may vary in different examples. For instance, the ultrasound sensor array 112 may include a 1D or 2D array of transducer elements, corresponding to linear array and matrix array probes, respectively. The 2D matrix array may be configured to scan electronically in both the elevational and azimuth dimensions (via phased array beamforming) for 2D or 3D imaging. In addition to B-mode imaging, imaging modalities implemented according to the disclosures herein can also include shear-wave and/or Doppler, for example.

A variety of users may handle and operate the ultrasound data acquisition unit 110 to perform the methods described herein. In some examples, the user may be an inexperienced, novice ultrasound operator unable to efficiently identify each anatomical or artificial feature required to be obtained in a given scan.

**FIG. 2** is a block diagram of an example collection of computational modules that may be embodied or implemented by the data processor 126 alone, or by one or more data processors operating concurrently or sequentially with the data processor 126. The modules may include an image-based probe position/orientation module 202 running at least one deep neural network 204, an image-based motion tracker module 206, a sensor-based probe position/orientation module 207, a sequence pattern analysis module 208, a log file generation module 210, and a narration module 212. The modules may be separate and distinct, as depicted, or two or more of the modules may be combined into a single module, circuitry, or processor. For instance, the image-based probe position/orientation module 202 and motion tracker module 206 may constitute a single module configured to perform multiple functions. Additional embodiments may feature one processor, e.g., a signal path processor, dedicated to running at least one deep neural network and/or recognizing ultrasound probe position, orientation and/or movement. One or more additional processors may be configured to generate log files documenting user actions during an ultrasound exam, identify notable user inputs received during the exam, and/or convert generated log files into narrative text descriptions.

The image-based probe position/orientation module 202 may be configured to implement at least one deep neural network 204 trained to determine and extract the position and orientation of an ultrasound probe relative to a subject being imaged during an ultrasound exam. In embodiments, the neural network 204 may be trained to identify various landmarks present within certain ultrasound images and/or image loops acquired during an ultrasound exam. Non-limiting examples of such landmarks may include anatomical features, e.g., organs, bones, etc., as well as characteristics thereof, e.g., the shape of the organs, the adjacency of two or more organs, etc. Additional landmarks recognized by the neural network 204 may include artificial features, such as ultrasound imaging artifacts (e.g., A- or B-lines), medical instruments, transducer markers, etc.

Using the identified landmark(s) present in a given image frame, the neural network 204 may determine the associated probe position/orientation used to acquire the image. At the onset of an exam, the neural network 204 output related to probe view/position/orientation may provide the initial context for a particular scan protocol, such that subsequent determinations of probe manipulation may be predicted and/or determined in view of the remaining steps required for the protocol. Once the initial imaging context has been estimated, one can "track" the probe's motion by performing frame-to-frame analysis and cumulatively adding together the estimated motion vectors. Other analytic or non-analytic (AI-based) methods of motion tracking are also possible. Such motion tracking can either increase the confidence of the initial context estimate, or, if the probe moves to a new location, it can extend/update the context to the new scanning area.

In some examples, the neural network 204 may be configured to operate on a frame-by-frame basis, such that probe position/orientation determinations may be made repeatedly in real or substantially real time throughout an ultrasound exam. Rapid operation of the neural network 204 in this manner may enable subtle changes in probe position/orientation to be detected, thereby improving the sensitivity of the system as a whole, which may be especially important for accurately capturing probe manipulations performed while examining relatively small target regions, such as the heart.

The image-based motion tracker module 206 may be configured to determine probe manipulations, e.g., adjustments in probe position and orientation, by comparing the determined position and orientation of the probe across multiple image frames using the frame-by-frame output generated by the neural network 204. In some examples, determination of the probe manipulations may be executed at least in part without analyzing the neural network output. For example, one or more signal processing techniques employed during the ultrasound scan may be used to initiate and/or perform the motion tracking. In addition or alternatively, the motion tracker module 206 may be configured to determine probe manipulations and the resulting probe positions/orientations by stringing together estimated motion vectors from one image frame to the next using the initial context provided by the neural network 204 as a starting point. By cumulatively adding motion vectors in this manner, information indicating how the user is navigating a scan protocol may be obtained without the use of external sensors, e.g., mounted cameras, electromagnetic sensors or optical trackers. Extraneous or inefficient probe manipulation may also be identified and flagged for subsequent review. Motion tracking can also increase the confidence of the initial context estimate or update and/or extend the context to a new scanning area upon movement of the probe.

Some embodiments may include a sensor-based probe position/orientation module 207 configured to augment or replace image-based module 202 and/or 206 by extracting probe position/orientation/motion using at least one external sensor coupled therewith. For embodiments in which the external sensor comprises a camera mounted in an exam room, the sensor module 207 may be configured to identify and track key anatomical features of the user handling the ultrasound probe to decipher user movement and thus probe manipulation over six degrees of freedom (6DoF). For instance, the user's arms, wrists and hands may all be related to probe manipulation. The external sensor may detect the position and movement of any one or all of these anatomical features of the user throughout an exam, which may then be utilized by the sensor-based probe position/orientation module 207 to determine corresponding probe positions/orientations/motions during the exam. The sensor-based probe position/orientation module 207 may also be configured to mask one or more features visible to the sensor, such as sensitive anatomical features of the subject not to be recorded due to privacy concerns. In some examples, the sensor-based probe position/orientation module 207 may also be configured to operate a deep neural network 209 trained to identify certain positions of the user and link those positions to corresponding image views and/or probe positions/orientations/motions.

The sequence pattern analysis module 208 may be configured to identify, filter, and select the most meaningful user inputs, e.g., button pushes and keystrokes, received by the imaging system during a given exam for inclusion in a final log file summary of the exam. Meaningful user inputs may include the inputs necessary for executing a particular scan protocol, including one or more maneuvers, steps, or acquisitions implemented in connection therewith. Meaningful inputs may also include inputs that may not be required for implementing a scan protocol, but which may be indicative of specific user actions that may be captured and recorded to accurately document notable details of user performance. For instance, meaningful inputs may include user inputs indicative of an incidental finding and subsequent examination thereof.

By way of illustration, particular sequences of buttons are often pushed for image optimization, quantification, and feature measurement during a given ultrasound exam. Abdominal imaging scans, for instance, commonly involve labelling of the kidney, aorta and inferior vena cava, as well as obtaining measurements feature length, width, height, circumference and/or stenosis. Each of the aforementioned actions may be associated with one or more meaningful user inputs. Color Doppler imaging, shear-wave elastography, and microflow imaging exams can also be initiated during an exam to obtain one or more of the noted measurements/labels. The initiation and termination of these additional imaging modes may also constitute meaningful user inputs. In specific embodiments, the sequence pattern analysis module 208 may be configured to extract *N* number of user inputs that collectively summarize the workflow related to image acquisition and final probe orientation. Extraction can be triggered based on a current image view and the remaining steps to be followed in accordance with a selected scan protocol. In some embodiments, the sequence pattern analysis module 208 may be configured to perform sequential pattern mining to pre-select the most frequent button usage patterns implemented by users during a given ultrasound scan based on real-time imaging context and clinical operation.

In addition to performing a sequence analysis to identify meaningful user inputs that describe or add value to the exam, the sequence pattern analysis module 208 may determine various indicators of user performance, such as the "percent usefulness" of a user's actions. For instance, the system may determine that 70% of a user's button pushes were essential to the exam, or that the user completed the exam with an average time-to-derived-view of *T* minutes, which may be faster or slower than X% of users performing the same exam on the same or similar subject. These determinations may be made by comparing the actual user inputs received during an exam to a set of pre-defined, expected inputs associated with a particular exam protocol. These and other similar statistics may be especially valuable for determining and monitoring changes in user performance over time. Similar performance statistics may also be used to modify one or more components of an imaging system and/or the protocols associated with certain exam types to improve workflow. Such adjustments may be made in response to common performance inefficiencies detected across a plurality of users.

The log file generation module 210 may be configured to pair the probe positions/orientations/movements determined by modules 202, 206 and/or 207 to log files of meaningful user inputs selected by the sequence pattern analysis module 208, such that the meaningful user inputs documented in the log files are augmented by the probe manipulation data. Pairing probe manipulation data to meaningful user inputs may be achieved in multiple ways. In some embodiments, for instance, the log file generation module 210 may be configured to create new object events in an existing XML/CDF workflow log file, where each new object event may document a probe view, position, orientation and/or motion corresponding to a particular timestamp. According to such embodiments, the log file generation module 210 may insert the newly created event into an existing log file of the associated user inputs having the same timestamp. In addition or alternatively, the log file generation module 210 may be configured to generate entirely new log files exclusively for probe position, orientation and motion that are then merged or synchronized via timestamp with the acquisition workflow embodied by the meaningful user inputs. Embodiments may thus involve obtaining or receiving a pre-determined scanning protocol, which may be associated with pre-defined user inputs necessary to execute the protocol, identifying meaningful user inputs from a current exam, pairing the meaningful user inputs to probe position/orientation data, and in some cases, comparing the paired data to the pre-determined scanning protocol.

Comprehensive log files documenting both probe manipulations and meaningful user inputs implemented during a given ultrasound exam may provide a detailed record of the exam for further review as well as a detailed set of instructions for users performing the same or similar exam in the future on the same or similar subject. Future users may, for example, use the log files associated with a given image to determine which user inputs and probe manipulations were necessary to obtain the image. The comprehensive log files may also facilitate radiologist review, in particular, which may be especially helpful for analyzing challenging cases associated with low image quality, e.g., cases involving high-BMI subjects.

Because the raw data embodying user inputs and probe manipulations documented in log files may not be particularly helpful to clinicians attempting to interpret exam workflow and performance, the narration module 212 may be configured to convert the log files into readable narratives of the exam. In some examples, user-friendly narratives may comprise a textual summary of the exam generated via natural language processing of the log file scripts. The written summary, which may include step-by-step narratives of discrete steps or user actions implemented during a particular scan, along with the most meaningful steps of the scan, may be easily read by a person reviewing the exam, such as a radiologist. The review made possible by operation of the narration module 212 may thus enable reviewers to determine whether a user followed a scan protocol and decipher whether incidental findings were obtained. Narratives generated for a particular exam may also provide improved guidance to future users performing the same exam by revealing how a previous user performed the exam, for example by showing what probe maneuvers were performed to acquire certain images. Given that, practically speaking, the same users cannot always perform the same exams of the same subjects, the instructions provided by the generated narratives may improve the accuracy of exam findings and conclusions drawn therefrom, while also increasing exam speed and efficiency. To convert the log files into narrative text, the narration module 212 may access and extract the log files from a storage memory, e.g., memory 140.

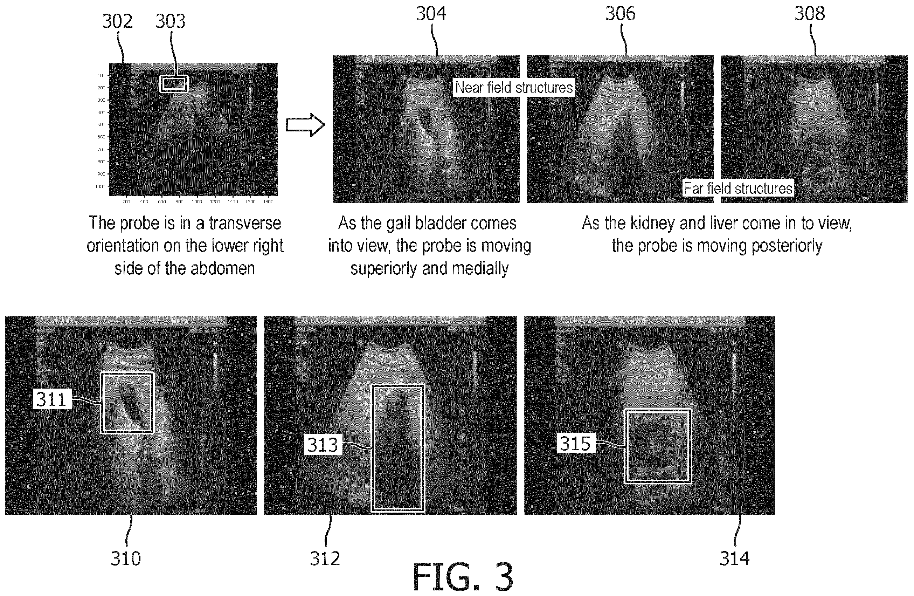

**FIGS. 3-7** comprise images, schematics, and sample log files illustrating various operations of the aforementioned modules, which as noted above, may each be separate and distinct or unified into one or more processors, e.g., data processor 126.

**FIG. 3** shows various image snapshots taken during an abdominal scan in accordance with embodiments disclosed herein. Image 302 includes a variety of features, one or more of which may be recognized by a deep neural network configured to output the position and orientation of the ultrasound probe used to acquire the image 302. In the particular snapshot shown, the system determines that given the features displayed in the image, including a transducer marker 303, the probe is in a transverse orientation on the lower right side of the abdomen.

The image 302 may be utilized by the system to determine the initial context of an ultrasound exam given the associated scan protocol received via user input or extracted by one or more processors in view of the exam type. In some examples, exam selection may comprise user selection of a tissue pre-set (TSP) indicating which part of the body is being imaged. By limiting the exam to a particular TSP, the search space to be analyzed by the underlying neural network(s) may be reduced, thereby facilitating navigation of the scan protocol and prediction of the likely user inputs, probe positions/orientations/motions and corresponding views used to perform the exam. The search space may vary significantly across different exam types. For instance, the search space is typically much larger for abdominal scans than for cardiac scans, and as a result, the types and degree of probe manipulations performed by a user may be drastically different between the two exams. Notably, embodiments may identify instances in which a TSP is incorrectly selected, for example by determining that the selected TSP does not align with a transducer marker, imaged anatomical feature, organ shape, and/or organ proximity. This issue can be flagged to the user in real time, for example via display of a graphic or alert on a user interface, such that the necessary adjustments to TSP selection or probe position/orientation can be made before beginning the exam. In addition or alternatively, this issue can be flagged for inclusion in the final log file record and narrative exam summary.

Moving to the right, images 304, 306, and 308 depict various features that may appear and be recognized by the image-based probe position/orientation module 202 and/or motion tracker module 206 as the probe is manipulated to perform the exam. The module(s) may determine, for instance, that as the gall bladder comes into view (image 304), the probe must be moving superiorly and medially. As the kidney and liver come in to view (images 306 and 308), the probe must be moving posteriorly. These determinations may be made by recognizing both near- and far-field structures present in the images, and because it is commonly observed that the near-field structures visible in each ultrasound image may change only slightly during abdominal exams, the deep neural network may be trained to focus on landmarks located deeper in the far field. As is evident from the displayed images, a variety of diverse features ranging from barely visible to hyperechoic may complicate the analysis of each image. As such, the automatic recognition of the specific features indicative of probe position/orientation/motion performed by the systems herein may be especially valuable.

In some examples, the system may be configured to select one of a plurality of deep neural networks given the selected exam protocol. Upfront selection of a network trained specifically to process images from a particular exam may improve the speed and accuracy of probe position/orientation/motion interpretation.

Images 310, 312, and 314 include bounding boxes 311, 313, 315 flagging certain landmarks that may be utilized and/or generated by a neural network or associated processor, and optionally displayed. The neural network(s) may be configured to identify the features present in a given bounding box or identify whether one or more landmarks from a subset of landmarks are present or not. The bounding boxes may be extracted to determine the presence and location of various landmarks, along with their locations relative to each in the image space. Cross-frame motion may then be evaluated, for example by the motion tracker module 206, by tracking the relative motion of the landmarks and thus the manipulations of the ultrasound probe used to image them. The particular landmarks and features thereof used for tracking may vary, and may comprise a mixture of anatomical features and ultrasound artifacts or effects. In some embodiments, relative motion may be determined by tracking speckle noise across a sequence of image frames.

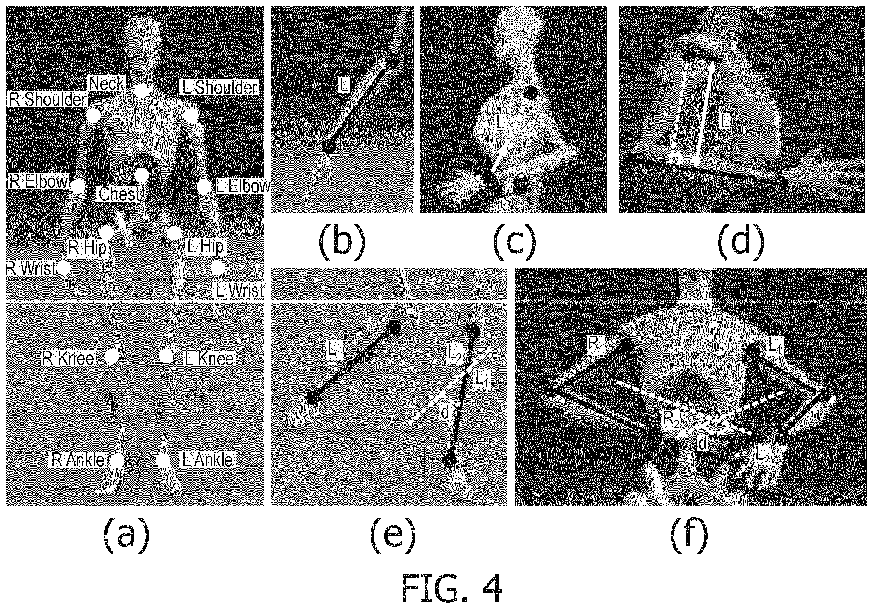

**FIG. 4** shows a variety of graphics illustrating examples of key points that may be detected and tracked using an external sensor, e.g., camera, mounted or otherwise positioned in an exam room. As shown in graphic (a), the sensor may be configured to recognize and monitor about 14 points on a user's body. It should be understood that this number is not limiting, as a variety of sensors may be configured to identify less than or greater than 14 points.

In the illustrated embodiment, the points are located on the user's neck, shoulders, elbows, wrists, chest, hips, knees and ankles. Graphics (b), (c), (d), (e), and (f) illustrate examples of the lengths, angles, and relative positions between various points that can be detected, measured and recorded during an exam to derive the probe manipulations related to the same. For instance, graphics (c) and (d) show that the distance between the user's same-side wrist and shoulder, along with the associated angle between the user's forearm and shoulder plane can be measured to determine probe position. Together, the absolute and relative measurements obtained via the sensor(s) can be utilized to determine and log probe position/orientation/motion with six degrees of freedom (6DoF). The sensor-based probe position/orientation module 207 may, in some examples, augment the determinations made by the image-based probe position/orientation module 202 and motion tracker module 206.

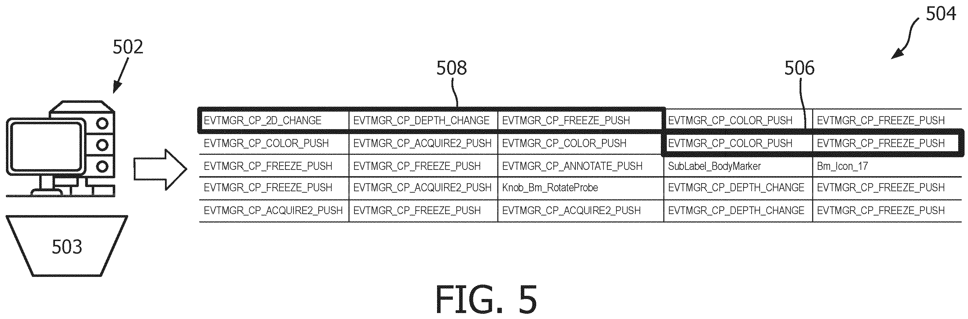

**FIG. 5** depicts the identification and selection of meaningful user inputs received during an imaging exam and logged in the example format shown. As illustrated, the system may first extract the exam protocol 502 corresponding to the exam being performed by the user, thereby also extracting or receiving a set of pre-defined, expected user inputs 503 associated with the protocol 502. Using the extracted exam protocol 502 and expected user inputs 503 as a template, the system may identify which of the plurality of actual user inputs 504 received during the exam were necessary for following the protocol, thereby distinguishing such meaningful inputs, e.g., user input 506, from those not necessarily required for performance of the exam, e.g., user input 508. As noted above, the sequence pattern analysis module 208 may, in specific embodiments, extract *N* meaningful user inputs, e.g., button pushes, for a given clinical application and workflow related to a specific scanning protocol.

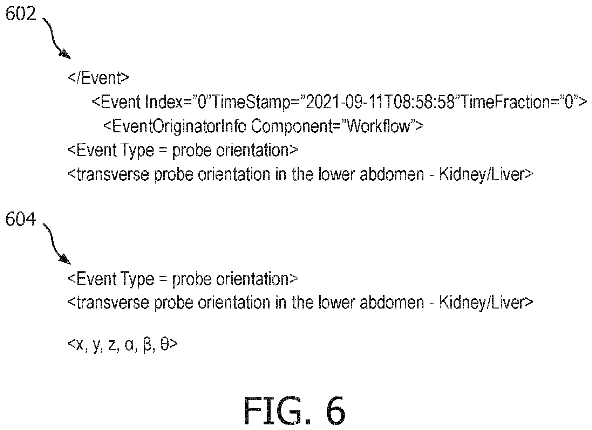

**FIG. 6** depicts examples of abbreviated log files generated in accordance with embodiments disclosed herein. Object event 602 represents a log file event documenting a probe position/orientation identified and timestamped for insertion into an existing workflow log file documenting corresponding user inputs. As shown, the particular event 602 is timestamped "2021-09-11T08:58:58." The event type is "probe orientation" described as "transverse probe orientation in the lower abdomen - kidney/liver."

If 6DoF are available via operation of at least one external sensor and associated processor, e.g., sensor-based probe position/orientation module 207, a log file event 604 documenting probe position/orientation may further specify "x, y, z, α, β, θ" dimensions.

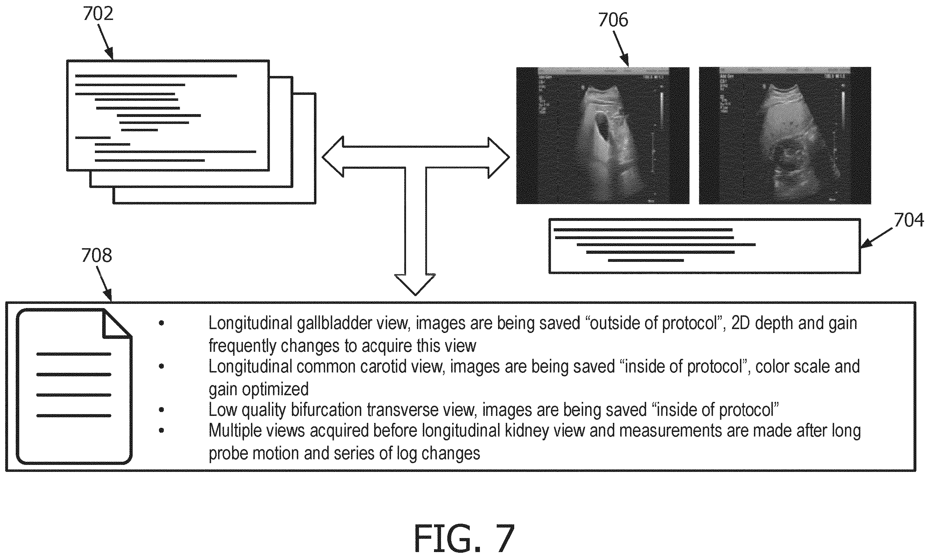

**FIG. 7** is a schematic representation of the use of workflow log files documenting the system usage and probe manipulations to generate a point-by-point narrative summary of an ultrasound exam. As shown, the workflow logs 702 documenting a plurality of meaningful user inputs can be integrated or synchronized with newly generated log files or object events 704 created by a disclosed system via landmark recognition and tracking of acquired ultrasound images 706 and/or external sensor-based tracking of a user performing an exam. The resulting comprehensive log files may then be converted into a narrative summary 708 of the exam, which includes descriptions of both user inputs and probe manipulations. The narrative summary 708 may include a list of acquired views and user actions, along with an indication of whether certain views and/or user actions are deemed inside or outside of the selected scan protocol. For instance, the first enumerated narrative reads "longitudinal gallbladder view, images are being saved 'outside of protocol', 2D depth and gain frequently changes to acquire this view." The second enumerated narrative reads "longitudinal common carotid view, images are being saved 'inside of protocol', color scale and gain optimized." The third enumerated narrative reads "low quality bifurcation transverse view, images are being saved 'inside of protocol'." The fourth narrative reads multiple views acquired before longitudinal kidney view and measurements are made after long probe motion and series of log changes." The narrative summary may be generated any time after the inventive log files are generated, including just after the exam or later.

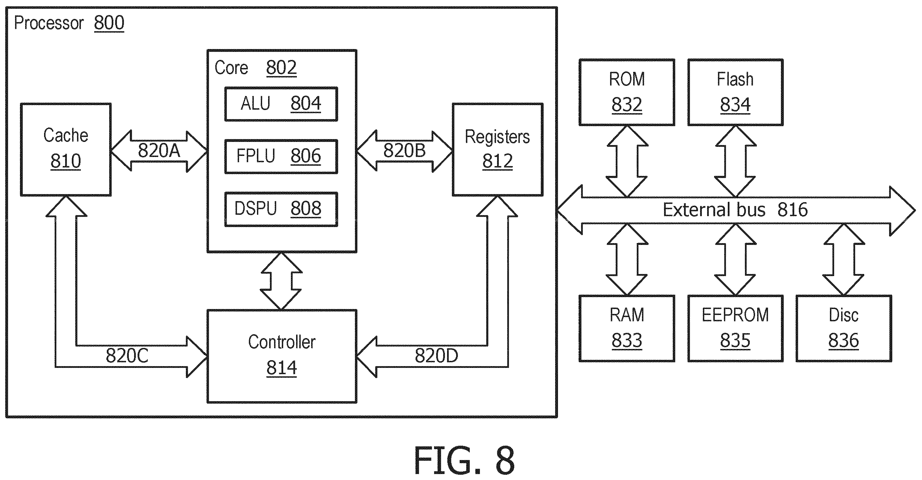

**FIG. 8** is a simplified block diagram illustrating an example processor 800 according to principles of the present disclosure. One or more processors utilized to implement the disclosed methods, such as processor 126 of system 100, may be configured the same as or similarly to processor 800.

Processor 800 may be any suitable processor type including, but not limited to, a microprocessor, a microcontroller, a digital signal processor (DSP), a field programmable array (FPGA) where the FPGA has been programmed to form a processor, a graphical processing unit (GPU), an application specific circuit (ASIC) where the ASIC has been designed to form a processor, or a combination thereof.

The processor 800 may include one or more cores 802. The core 802 may include one or more arithmetic logic units (ALU) 804. In some examples, the core 802 may include a floating point logic unit (FPLU) 806 and/or a digital signal processing unit (DSPU) 808 in addition to or instead of the ALU 804.

The processor 800 may include one or more registers 812 communicatively coupled to the core 802. The registers 812 may be implemented using dedicated logic gate circuits (e.g., flip-flops) and/or any memory technology. In some examples the registers 812 may be implemented using static memory. The register may provide data, instructions and addresses to the core 802.

In some examples, processor 800 may include one or more levels of cache memory 810 communicatively coupled to the core 802. The cache memory 810 may provide computer-readable instructions to the core 802 for execution. The cache memory 810 may provide data for processing by the core 802. In some examples, the computer-readable instructions may have been provided to the cache memory 810 by a local memory, for example, local memory attached to the external bus 816. The cache memory 810 may be implemented with any suitable cache memory type, for example, metal-oxide semiconductor (MOS) memory such as static random access memory (SRAM), dynamic random access memory (DRAM), and/or any other suitable memory technology.

The processor 800 may include a controller 814, which may control input to one or more processors included herein, e.g., processor 126. Controller 814 may control the data paths in the ALU 804, FPLU 806 and/or DSPU 808. Controller 814 may be implemented as one or more state machines, data paths and/or dedicated control logic. The gates of controller 814 may be implemented as standalone gates, FPGA, ASIC or any other suitable technology.

The registers 812 and the cache memory 810 may communicate with controller 814 and core 802 via internal connections 820A, 820B, 820C and 820D. Internal connections may implemented as a bus, multiplexor, crossbar switch, and/or any other suitable connection technology.

Inputs and outputs for the processor 800 may be provided via a bus 816, which may include one or more conductive lines. The bus 816 may be communicatively coupled to one or more components of processor 800, for example the controller 814, cache 810, and/or register 812. The bus 816 may be coupled to one or more components of the system.

The bus 816 may be coupled to one or more external memories. The external memories may include Read Only Memory (ROM) 832. ROM 832 may be a masked ROM, Electronically Programmable Read Only Memory (EPROM) or any other suitable technology. The external memory may include Random Access Memory (RAM) 833. RAM 833 may be a static RAM, battery backed up static RAM, Dynamic RAM (DRAM) or any other suitable technology. The external memory may include Electrically Erasable Programmable Read Only Memory (EEPROM) 835. The external memory may include Flash memory 834. The external memory may include a magnetic storage device such as disc 836.

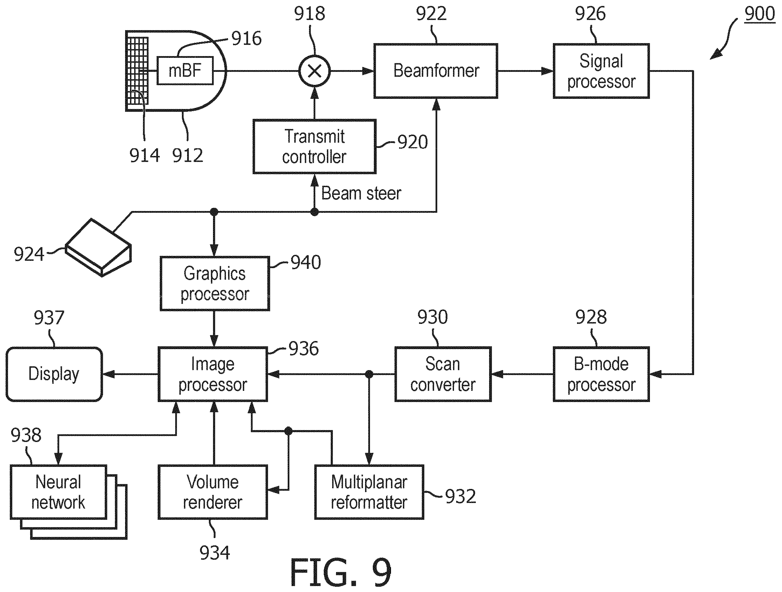

**FIG. 9** is a block diagram of another ultrasound system 900 in accordance with principles of the present disclosure. One or more components shown in **FIG. 9** may be included within a system configured to determine and track a series of probe manipulations and user inputs implemented during a particular ultrasound exam. One or more of the components may also be configured to generate log files documenting the tracked manipulations and user inputs. For example, one or more of the above-described functions of data processor 126 and/or ultrasound data acquisition unit 110 may be implemented and/or controlled by one or more of the processing components shown in **FIG. 9****,** including for example, signal processor 926, B-mode processor 928, scan converter 930, multiplanar reformatter 932, volume renderer 934 and/or image processor 936.

In the system of **FIG. 9****,** an ultrasound probe 912 includes a transducer array 914 for transmitting ultrasonic waves into a targeted region of a subject pursuant to a scan protocol and receiving echo information responsive to the transmitted waves. In various embodiments, the transducer array 914 may be a matrix array or a one-dimensional linear array. The transducer array 914 may be coupled to a microbeamformer 916 in the probe 912, which may control the transmission and reception of signals by the transducer elements in the array. In the example shown, the microbeamformer 916 is coupled by the probe cable to a transmit/receive (T/R) switch 918, which switches between transmission and reception and protects the main beamformer 922 from high energy transmit signals. In some embodiments, the T/R switch 918 and other elements in the system can be included in the transducer probe rather than in a separate ultrasound system component. The transmission of ultrasonic beams from the transducer array 914 under control of the microbeamformer 916 may be directed by the transmit controller 920 coupled to the T/R switch 918 and the beamformer 922, which receives input, e.g., from the user's operation of the user interface or control panel 924. A function that may be controlled by the transmit controller 920 is the direction in which beams are steered. Beams may be steered straight ahead from (orthogonal to) the transducer array, or at different angles for a wider field of view. The partially beamformed signals produced by the microbeamformer 916 are coupled to a main beamformer 922 where partially beamformed signals from individual patches of transducer elements are combined into a fully beamformed signal.

The beamformed signals may be communicated to a signal processor 926. The signal processor 926 may process the received echo signals in various ways, such as bandpass filtering, decimation, I and Q component separation, and/or harmonic signal separation. The signal processor 926 may also perform additional signal enhancement via speckle reduction, signal compounding, and/or noise elimination. In some examples, data generated by the different processing techniques employed by the signal processor 926 may be used by a data processor and/or at least one neural network. The processed signals may be coupled to a B-mode processor 928, which may employ amplitude detection for imaging structures in the body. The signals produced by the B-mode processor 928 may be coupled to a scan converter 930 and a multiplanar reformatter 932. The scan converter 930 may arrange the echo signals in the spatial relationship from which they were received in a desired image format. For instance, the scan converter 930 may arrange the echo signals into a two dimensional (2D) sector-shaped format. The multiplanar reformatter 932 may convert echoes which are received from points in a common plane in a volumetric region of the body into an ultrasonic image of that plane, as described in U.S. Pat. No. 6,443,896 (Detmer). In some examples, a volume renderer 934 may convert the echo signals of a 3D data set into a projected 3D image as viewed from a given reference point, e.g., as described in U.S. Pat. No. 6,530,885 (Entrekin et al.). The 2D or 3D images may be communicated from the scan converter 930, multiplanar reformatter 932, and volume renderer 934 to an image processor 936 for further enhancement, buffering and/or temporary storage for display on an image display 937.

A graphics processor 940 may generate graphic overlays for display with the processed ultrasound images. These graphic overlays may contain, for example, standard identifying information such as subject name, date and time of the image, imaging parameters, and the like. Overlays may also include one or more indicators of probe position, orientation and/or manipulation generated by at least one neural network 938 in connection with a particular image frame. In some examples, the graphics processor 940 may receive input from the user interface 924, such as a typed patient name or confirmation that an instruction displayed or emitted from the interface has been acknowledged and/or implemented by the user of the system 900. The user interface 924 may also receive input regarding subject characteristics, the selection of particular imaging modalities and the operating parameters included in such modalities, input prompting adjustments to the settings and/or parameters used by the system 900, input requesting additional instructions or assistance for performing an ultrasound scan, and/or input requesting that one or more ultrasound images be saved and/or transmitted to a remote receiver. The user interface 924 may also be coupled to the multiplanar reformatter 932 for selection and control of a display of multiple multiplanar reformatted (MPR) images.

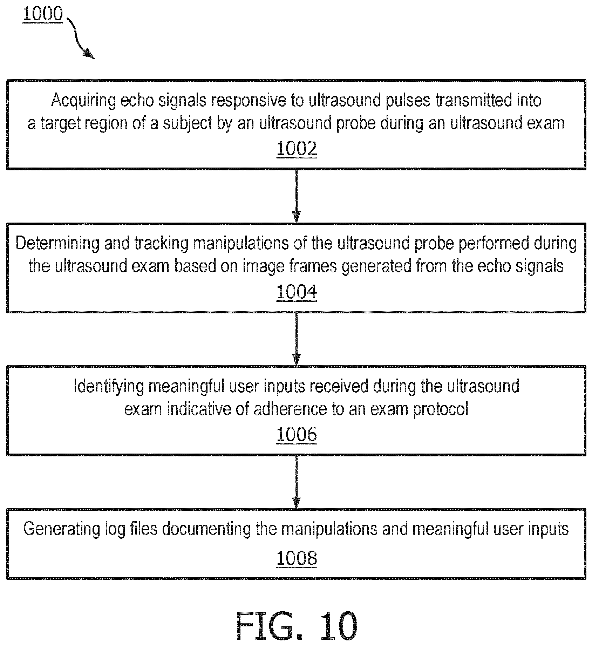

**FIG. 10** is a flowchart illustrating an example method of ultrasound imaging that involves determining and tracking probe manipulation and received user inputs during a given ultrasound exam. The example method 1000 shows the steps that may be utilized, in any sequence, by the systems and/or apparatuses described herein for performing the method, which may further involve storing the probe manipulation data together with the user inputs in comprehensive log files and converting the log files into narrative text for improved readability. The method 1000 may be performed by an ultrasound imaging system, such as system 100, or other systems including, for example, a mobile system such as LUMIFY by Koninklijke Philips N.V. ("Philips"). Additional example systems may include SPARQ and/or EPIQ, also produced by Philips.

In the embodiment shown, the method begins at block 1002 by "acquiring echo signals responsive to ultrasound pulses transmitted into a target region of a subject by an ultrasound probe during an ultrasound exam."

At block 1004, the method involves "determining and tracking manipulations of the ultrasound probe performed during the ultrasound exam based on image frames generated from the echo signals."

At block 1006, the method involves "identifying user inputs received during the ultrasound exam indicative of adherence to an exam protocol."

At block 1008, the method involves "generating log files documenting the manipulations and user inputs."

Of course, it is to be appreciated that any one of the examples, embodiments or processes described herein may be combined with one or more other examples, embodiments and/or processes, or be separated and/or performed amongst separate devices or device portions in accordance with the present systems, devices and methods.

In various embodiments where components, systems and/or methods are implemented using a programmable device, such as a computer-based system or programmable logic, it should be appreciated that the above-described systems and methods can be implemented using any of various known or later developed programming languages, such as "C", "C++", "FORTRAN", "Pascal", "VHDL" and the like. Accordingly, various storage media, such as magnetic computer disks, optical disks, electronic memories and the like, can be prepared that can contain information that can direct a device, such as a computer, to implement the above-described systems and/or methods. Once an appropriate device has access to the information and programs contained on the storage media, the storage media can provide the information and programs to the device, thus enabling the device to perform functions of the systems and/or methods described herein. For example, if a computer disk containing appropriate materials, such as a source file, an object file, an executable file or the like, were provided to a computer, the computer could receive the information, appropriately configure itself and perform the functions of the various systems and methods outlined in the diagrams and flowcharts above to implement the various functions. That is, the computer could receive various portions of information from the disk relating to different elements of the above-described systems and/or methods, implement the individual systems and/or methods and coordinate the functions of the individual systems and/or methods described above.

Finally, the above-discussion is intended to be merely illustrative of the present system and should not be construed as limiting the appended claims to any particular embodiment or group of embodiments. Thus, while the present system has been described in particular detail with reference to exemplary embodiments, it should also be appreciated that numerous modifications and alternative embodiments may be devised by those having ordinary skill in the art without departing from the broader and intended scope of the present system as set forth in the claims that follow. Accordingly, the specification and drawings are to be regarded in an illustrative manner and are not intended to limit the scope of the appended claims.

## Claims

1. An ultrasound imaging system, comprising:
an ultrasound probe (111) configured to acquire echo signals (118) responsive to ultrasound pulses (114) transmitted toward a subject during an ultrasound exam;
an input of pre-defined user inputs (503) associated with an exam protocol (502) that is similar to the ultrasound exam; and
one or more processors (126) in communication with the ultrasound probe and configured to:
determine and track manipulations of the ultrasound probe performed during the ultrasound exam based on image frames (124) generated from the echo signals;
identify meaningful user inputs (506) received during the ultrasound exam indicative of adherence to the exam protocol; and
generate log files (702, 704) documenting the manipulations and meaningful user inputs.

2. The system of claim 1, wherein the meaningful user inputs comprise:
an adjustment in at least one image acquisition setting comprising one or more of:
a gain, a scan depth, an image contrast level, a color scale, a probe type, a frequency, or an imaging mode; or
one or more of: an image snapshot, a label, or a measurement of one or more features present in the image frames.

3. The system of claim 1, wherein the manipulations of the ultrasound probe comprise changes in probe position and orientation.

4. The system of claim 1, wherein the one or more processors are configured to determine and track manipulations of the ultrasound probe by inputting the image frames to a neural network, the neural network trained to:
identify at least one landmark present in one or more of the image frames; and
determine one or more ultrasound probe orientations and/or positions used to obtain the one or more image frames based on the at least one landmark identified therein,
wherein the at least one landmark optionally comprises one or more of: an anatomical feature, an image artifact, a medical instrument, or a transducer marker.

5. The system of claim 1, wherein the one or more processors are configured to:
identify meaningful user inputs received during the ultrasound exam indicative of adherence to an exam protocol by comparing a total list of actual user inputs to a list of expected user inputs associated with the exam protocol; of
generate narrative text describing the manipulations and meaningful user inputs documented in the log files.

6. The system of claim 1, wherein the one or more processors are configured to generate log files documenting the manipulations and meaningful user inputs by:
generating a first log file documenting the meaningful user inputs;
generating a second log file documenting the manipulations of the ultrasound probe; and
synchronizing the first log file with the second log file.

7. The system of claim 1, wherein the one or more processors are configured to generate log files documenting the manipulations and meaningful user inputs by:
generating a first log file documenting the meaningful user inputs;
generating one or more object events documenting the manipulations of the ultrasound probe; and
inserting the one or more object events into the first log file based on a unique timestamp associated with each of the one or more object events.

8. The system of claim 1, wherein the one or more processors are further configured to determine and track manipulations of the ultrasound probe based input received from an external sensor monitoring movements of a user handling the ultrasound probe.

9. The system of claim 8, wherein the one or more processors are configured to determine and track manipulations of the ultrasound probe by inputting the input received from the external sensor to a neural network, the neural network trained to:
identify one or more of a relative position, orientation, or angle between two or more anatomical features of the user; and
determine one or more ultrasound probe orientations and/or positions corresponding to the at least one relative position or angle.

10. A method of ultrasound imaging, the method comprising:
acquiring echo signals (118) responsive to ultrasound pulses (114) transmitted into a target region (116) of a subject by an ultrasound probe (111) during an ultrasound exam;
receiving input of pre-defined user inputs (503) associated with an exam protocol (502) that is similar to the ultrasound exam;
determining and tracking manipulations of the ultrasound probe performed during the ultrasound exam based on image frames (124) generated from the echo signals;
identifying meaningful user (506) inputs received during the ultrasound exam indicative of adherence to the exam protocol; and
generating log files (702, 704) documenting the manipulations and meaningful user inputs.

11. The method of claim 10,
wherein the meaningful user inputs comprise an adjustment in at least one image acquisition setting comprising one or more of: a gain, a scan depth, an image contrast level, a color scale, a probe type, a frequency, or an imaging mode; or
wherein the manipulations of the ultrasound probe comprise changes in probe position and orientation.

12. The method of claim 10, wherein determining and tracking manipulations of the ultrasound probe comprises identifying at least one landmark present in one or more of the image frames and determining one or more ultrasound probe orientations and/or positions used to obtain the one or more image frames based on the at least one landmark identified therein, and
wherein the at least one landmark optionally comprises one or more of: an anatomical feature, an image artifact, a medical instrument, or a transducer marker.

13. The method of claim 10, wherein generating log files documenting the manipulations and meaningful user inputs comprises:
generating a first log file documenting the meaningful user inputs;
generating a second log file documenting the manipulations of the ultrasound probe; and
synchronizing the first log file with the second log file.

14. The method of claim 10, wherein generating log files documenting the manipulations and meaningful user inputs comprises:
generating a first log file documenting the meaningful user inputs;
generating one or more object events documenting the manipulations of the ultrasound probe; and
inserting the one or more object events into the first log file based on a unique timestamp associated with each of the one or more object events.

15. A non-transitory computer-readable medium comprising executable instructions, which when executed cause a processor of a system to perform any of the methods of claims 10-14.

## Patentansprüche

1. Ultraschallbildgebungssystem, umfassend
eine Ultraschallsonde (111), die so konfiguriert ist, dass sie Echosignale (118) erfasst, die auf Ultraschallimpulse (114) reagieren, die während einer Ultraschalluntersuchung auf den Patienten gerichtet sind;
eine Eingabe vordefinierter Benutzereingaben (503), die mit einem Untersuchungsprotokoll (502) verknüpft sind, das der Ultraschalluntersuchung ähnelt; und
einen oder mehrere Prozessoren (126), die mit der Ultraschallsonde kommunizieren und konfiguriert sind zum:
Bestimmen und Verfolgen von Manipulationen der Ultraschallsonde, die während der Ultraschalluntersuchung durchgeführt werden, anhand von Bildrahmen (124), die aus den Echosignalen erzeugt werden;
Identifizieren aussagekräftiger Benutzereingaben (506), die während der Ultraschalluntersuchung empfangen werden und auf die Einhaltung des Untersuchungsprotokolls hinweisen; und
Erstellen von Protokolldateien (702, 704), die die Manipulationen und aussagekräftigen Benutzereingaben dokumentieren.

2. System nach Anspruch 1, wobei die aussagekräftigen Benutzereingaben Folgendes umfassen:
eine Anpassung mindestens einer Bildaufnahmeeinstellung, die eines oder mehrere der folgenden Elemente umfasst:
eine Verstärkung, eine Scantiefe, einen Bildkontrastpegel, eine Farbskala, einen Sondentyp, eine Frequenz oder einen Bildgebungsmodus; oder
eines oder mehrere der folgenden Elemente: eine Momentaufnahme des Bildes, eine Beschriftung oder eine Messung eines oder mehrerer Merkmale, die in den Bildrahmen vorhanden sind.

3. System nach Anspruch 1, wobei die Manipulationen der Ultraschallsonde Änderungen der Sondenposition und -orientierung umfassen.

4. System nach Anspruch 1, wobei der eine oder die mehreren Prozessoren so konfiguriert sind, dass sie Manipulationen der Ultraschallsonde durch Eingabe der Bildrahmen in ein neuronales Netzwerk bestimmen und verfolgen, wobei das neuronale Netzwerk darauf trainiert ist, Folgendes auszuführen:
Identifizieren mindestens eines markanten Merkmals, in einem oder mehreren der Bildrahmen; und
Bestimmen einer oder mehrerer Orientierungen und/oder Positionen der Ultraschallsonde, die verwendet wurden, um den einen oder die mehreren Bildrahmen auf der Grundlage des mindestens einen darin identifizierten markanten Merkmals zu erhalten,
wobei das mindestens eine markante Merkmal optional eines oder mehrere der folgenden Elemente umfasst: ein anatomisches Merkmal, ein Bildartefakt, ein medizinisches Instrument oder eine Wandlermarkierung.

5. System nach Anspruch 1, wobei der eine oder die mehreren Prozessoren konfiguriert sind zum:
Identifizieren aussagekräftiger Benutzereingaben, empfangen während der Ultraschalluntersuchung, die auf die Einhaltung des Untersuchungsprotokolls hinweisen, durch Vergleichen einer Gesamtliste von tatsächlichen Benutzereingaben mit einer Liste der dem Untersuchungsprotokoll zugeordneten Benutzereingaben; oder
Erstellen eines narrativen Texts, der die in den Protokolldateien dokumentierten Manipulationen und aussagekräftigen Benutzereingaben beschreibt.

6. System nach Anspruch 1, wobei der eine oder die mehreren Prozessoren so konfiguriert sind, dass sie Protokolldateien generieren, die die Manipulationen und aussagekräftigen Benutzereingaben dokumentieren, durch:
Erstellen einer ersten Protokolldatei, die die aussagekräftigen Benutzereingaben dokumentiert;
Erstellen einer zweiten Protokolldatei, die die Manipulationen der Ultraschallsonde dokumentiert; und Synchronisieren der ersten Protokolldatei mit der zweiten Protokolldatei.

7. System nach Anspruch 1, wobei der eine oder die mehreren Prozessoren so konfiguriert sind, dass sie Protokolldateien generieren, die die Manipulationen und aussagekräftigen Benutzereingaben dokumentieren, durch:
Erstellen einer ersten Protokolldatei, die die aussagekräftigen Benutzereingaben dokumentiert;
Erstellen eines oder mehrerer Objektereignisse, die die Manipulationen der Ultraschallsonde dokumentieren; und
Einfügen des einen oder der mehreren Objektereignisse in die erste Protokolldatei basierend auf einem eindeutigen Zeitstempel, der jedem der einen oder mehreren Objektereignisse zugeordnet ist.

8. System nach Anspruch 1, wobei der eine oder die mehreren Prozessoren weiter so konfiguriert sind, dass sie Manipulationen der Ultraschallsonde auf der Grundlage von Eingaben ermitteln und verfolgen, die von einem externen Sensor empfangen werden, der die Bewegungen eines Benutzers überwacht, der die Ultraschallsonde handhabt.

9. System nach Anspruch 8, wobei der eine oder die mehreren Prozessoren so konfiguriert sind, dass sie Manipulationen der Ultraschallsonde durch Eingabe der vom externen Sensor empfangenen Eingabe in ein neuronales Netzwerk bestimmen und verfolgen, wobei das neuronale Netzwerk darauf trainiert ist, Folgendes durchzuführen:
Identifizieren eines oder mehrerer von einer relativen Position, Orientierung oder Winkel zwischen zwei oder mehr anatomischen Merkmalen des Benutzers; und
Bestimmen einer oder mehrerer Orientierungen und/oder Positionen der Ultraschallsonde, die mindestens einer relativen Position oder einem relativen Winkel entsprechen.

10. Verfahren zur Ultraschallbildgebung, wobei das Verfahren Folgendes umfasst:
Erfassen von Echosignalen (118), die auf Ultraschallimpulse (114) reagieren, welche mit einer Ultraschallsonde (111) während einer Ultraschalluntersuchung in einen Zielbereich (116) eines Patienten übertragen werden;
Empfangen von vordefinierten Benutzereingaben (503) im Zusammenhang mit einem Untersuchungsprotokoll (502), das der Ultraschalluntersuchung ähnelt;
Bestimmen und Verfolgen von Manipulationen der Ultraschallsonde während der Ultraschalluntersuchung anhand von Bildrahmen (124), die aus den Echosignalen erzeugt werden;
Identifizieren aussagekräftiger Benutzereingaben (506), die während der Ultraschalluntersuchung empfangen werden und auf die Einhaltung des Untersuchungsprotokolls hinweisen; und
Erstellen von Protokolldateien (702, 704), die die Manipulationen und aussagekräftigen Benutzereingaben dokumentieren.

11. Verfahren nach Anspruch 10,
wobei die aussagekräftigen Benutzereingaben eine Anpassung mindestens einer Bildaufnahmeeinstellung umfassen, die eine oder mehrere der folgenden Eigenschaften beinhaltet: Verstärkung, Scantiefe, Bildkontraststufe, Farbskala, Sondentyp, Frequenz oder Bildgebungsmodus; oder
wobei die Manipulationen der Ultraschallsonde Änderungen der Sondenposition und -orientierung umfassen.

12. Verfahren nach Anspruch 10, wobei Bestimmen und Verfolgen von Manipulationen der Ultraschallsonde Identifizieren mindestens eines in einem oder mehreren Bildrahmen vorhandenen markanten Merkmals und Bestimmen einer oder mehrerer Orientierungen und/oder Positionen der Ultraschallsonde umfasst, die verwendet wurden, um die ein oder mehreren Bildrahmen basierend auf dem mindestens einen darin identifizierten markanten Merkmal zu erhalten, und
wobei das mindestens eine markante Merkmal optional eines oder mehrere der folgenden Elemente umfasst: ein anatomisches Merkmal, ein Bildartefakt, ein medizinisches Instrument oder eine Wandlermarkierung.

13. Verfahren nach Anspruch 10, wobei Erstellen von Protokolldateien, die die Manipulationen und aussagekräftigen Benutzereingaben dokumentieren, Folgendes umfasst:
Erstellen einer ersten Protokolldatei, die die aussagekräftigen Benutzereingaben dokumentiert;
Erstellen einer zweiten Protokolldatei, die die Manipulationen der Ultraschallsonde dokumentiert; und
Synchronisieren der ersten Protokolldatei mit der zweiten Protokolldatei.

14. Verfahren nach Anspruch 10, wobei Erstellen von Protokolldateien, die die Manipulationen und aussagekräftigen Benutzereingaben dokumentieren, Folgendes umfasst:
Erstellen einer ersten Protokolldatei, die die aussagekräftigen Benutzereingaben dokumentiert;
Erstellen eines oder mehrerer Objektereignisse, die die Manipulationen der Ultraschallsonde dokumentieren; und
Einfügen des einen oder der mehreren Objektereignisse in die erste Protokolldatei basierend auf einem eindeutigen Zeitstempel, der jedem der einen oder mehreren Objektereignisse zugeordnet ist.

15. Nichtflüchtiges, computerlesbares Medium, das ausführbare Anweisungen umfasst, die, wenn sie ausgeführt werden, einen Prozessor eines Systems veranlassen, eines der Verfahren der Ansprüche 10-14 durchzuführen.

## Revendications

1. Système d'imagerie par ultrasons, comprenant :
une sonde à ultrasons (111) configurée pour acquérir des signaux d'écho (118) en réponse aux impulsions ultrasonores (114) transmises vers un sujet au cours d'un examen par échographie ;
une entrée de données utilisateur prédéfinies (503) associée à un protocole d'examen (502) similaire à l'examen échographique ; et
un ou plusieurs processeurs (126) en communication avec la sonde à ultrasons et configurés pour :
déterminer et suivre les manipulations de la sonde à ultrasons effectuées pendant l'examen par échographie sur la base des images (124) générées à partir des signaux d'écho ;
identifier les entrées utilisateur significatives (506) reçues pendant l'examen d'échographie, indiquant le respect du protocole d'examen ; et
générer des fichiers journaux (702, 704) documentant les manipulations et les entrées significatives de l'utilisateur.

2. Système selon la revendication 1, dans lequel les entrées utilisateur significatives comprennent :
un réglage dans au moins un paramètre d'acquisition d'images comprenant un ou plusieurs des éléments suivants:
un gain, une profondeur de balayage, un niveau de contraste d'image, une échelle de couleurs, un type de sonde, une fréquence ou un mode d'imagerie ; ou
un ou plusieurs des éléments suivants : une capture d'écran, une étiquette ou une mesure d'une ou plusieurs caractéristiques présentes dans les images.

3. Système selon la revendication 1, dans lequel les manipulations de la sonde à ultrasons comprennent des changements de position et d'orientation de la sonde.

4. Système selon la revendication 1, dans lequel ledit un ou les processeurs sont configurés pour déterminer et suivre les manipulations de la sonde à ultrasons en fournissant les trames d'images à un réseau neuronal, le réseau neuronal étant entraîné à :
identifier au moins un point de repère présent dans une ou plusieurs images ; et
déterminer une ou plusieurs orientations de la sonde à ultrasons et/ou des positions utilisées pour obtenir la ou les images fixes en fonction du au moins un point de repère identifié dans celles-ci,
dans lequel le au moins un point de repère comprend optionnellement un ou plusieurs des éléments suivants : une caractéristique anatomique, un artefact d'image, un instrument médical ou un marqueur de transducteur.

5. Système selon la revendication 1, dans lequel ledit un ou les plusieurs processeurs sont configurés pour :
identifier les entrées utilisateur significatives reçues pendant l'examen échographique, indicatives du respect du protocole d'examen, en comparant une liste totale des entrées utilisateur réelles à une liste des entrées utilisateur attendues associées au protocole d'examen ; ou
générer un texte narratif décrivant les manipulations et les entrées utilisateur significatives documentées dans les fichiers journaux.

6. Système selon la revendication 1, dans lequel ledit un ou les plusieurs processeurs sont configurés pour générer des fichiers journaux documentant les manipulations et les entrées utilisateur significatives par :
la génération d'un premier fichier journal documentant les entrées utilisateur significatives ;
la génération d'un second fichier journal documentant les manipulations de la sonde à ultrasons ; et la synchronisation du premier fichier journal avec le second fichier journal.

7. Système selon la revendication 1, dans lequel ledit un ou les plusieurs processeurs sont configurés pour générer des fichiers journaux documentant les manipulations et les entrées utilisateur significatives par :
la génération d'un premier fichier journal documentant les entrées utilisateur significatives ;
la génération d'un ou de plusieurs événements d'objet documentant les manipulations de la sonde à ultrasons ; et
l'insertion dudit un ou des plusieurs événements d'objet dans le premier fichier journal en fonction d'un horodatage unique associé à chacun des événements d'objet.

8. Système selon la revendication 1, dans lequel ledit un ou les plusieurs processeurs sont en outre configurés pour déterminer et suivre les manipulations de la sonde à ultrasons en fonction des données reçues d'un capteur externe surveillant les mouvements d'un utilisateur manipulant la sonde à ultrasons.

9. Système selon la revendication 8, dans lequel ledit un ou les plusieurs processeurs sont configurés pour déterminer et suivre les manipulations de la sonde à ultrasons en transmettant les données reçues du capteur externe à un réseau neuronal, le réseau neuronal étant entraîné à :
identifier une ou plusieurs d'une position relative, d'une orientation ou d'un angle relatif entre deux ou plusieurs caractéristiques anatomiques de l'utilisateur ; et
déterminer une ou plusieurs orientations de la sonde à ultrasons et/ou des positions correspondant à la au moins une position ou à un angle relatif.

10. Procédé d'imagerie par ultrasons, le procédé comprenant :
l'acquisition de signaux d'écho (118) en réponse aux impulsions ultrasonores (114) transmises dans une région cible (116) d'un sujet par une sonde à ultrasons (111) au cours d'un examen d'échographie ;
la réception d'entrées utilisateur prédéfinies (503) associées à un protocole d'examen (502) similaire à l'examen par échographie ;
la détermination et le suivi des manipulations de la sonde à ultrasons effectuées pendant l'examen par échographie sur la base des images (124) générées à partir des signaux d'écho ;
l'identification des entrées significatives de l'utilisateur (506) reçues pendant l'examen échographique, indiquant le respect du protocole d'examen ; et
la génération de fichiers journaux (702, 704) documentant les manipulations et les entrées significatives de l'utilisateur.

11. Procédé selon la revendication 10,
dans lequel les entrées utilisateur significatives comprennent un réglage dans au moins un paramètre d'acquisition d'image comprenant un ou plusieurs des éléments suivants : un gain, une profondeur de balayage, un niveau de contraste de l'image, une échelle de couleurs, un type de sonde, une fréquence ou un mode d'imagerie ; ou
dans lequel les manipulations de la sonde à ultrasons comprennent des changements de position et d'orientation de la sonde.

12. Procédé selon la revendication 10, dans lequel la détermination et le suivi des manipulations de la sonde à ultrasons comprennent l'identification d'au moins un point de repère présent dans une ou plusieurs trames d'images et la détermination d'une ou plusieurs orientations de la sonde à ultrasons et/ou de positions utilisées pour obtenir ladite une ou les plusieurs trames d'images sur la base du au moins un point de repère identifié dans celles-ci, et
dans lequel le au moins un point de repère comprend optionnellement un ou plusieurs des éléments suivants : une caractéristique anatomique, un artefact d'image, un instrument médical ou un marqueur de transducteur.

13. Procédé selon la revendication 10, dans lequel la génération de fichiers journaux documentant les manipulations et les entrées utilisateur significatives comprend :
la génération d'un premier fichier journal documentant les entrées utilisateur significatives ;
la génération d'un second fichier journal documentant les manipulations de la sonde à ultrasons ; et
la synchronisation du premier fichier journal avec le second fichier journal.

14. Procédé selon la revendication 10, dans lequel la génération de fichiers journaux documentant les manipulations et les entrées utilisateur significatives comprend :
la génération d'un premier fichier journal documentant les entrées utilisateur significatives ;
la génération d'un ou plusieurs événements d'objet documentant les manipulations de la sonde à ultrasons ; et
l'insertion d'un ou plusieurs événements d'objet dans le premier fichier journal en fonction d'un horodatage unique associé à chacun des événements d'objet.

15. Support non transitoire lisible par ordinateur comprenant des instructions exécutables qui, lorsqu'elles sont exécutées, amènent un processeur d'un système à exécuter l'un quelconque des procédés selon les revendications 10-14.
